(19) Europäisches Patentamt
European Patent Office
Office européen des brevets

(11) Veröffentlichungsnummer: **0 089 640**
**B1**

# (12) EUROPÄISCHE PATENTSCHRIFT

(45) Veröffentlichungstag der Patentschrift: **17.09.86**

(51) Int. Cl.⁴: **C 12 N 9/04,** C 12 Q 1/32 // C12R1/01

(21) Anmeldenummer: **83102704.0**

(22) Anmeldetag: **18.03.83**

## (54) L(+)-Tartrat-Dehydrogenase, Verfahren zu deren Herstellung sowie Verfahren und Reagens zur Bestimmung von L(+)-Tartrat und D(+)-Malat.

(30) Priorität: **23.03.82 DE 3210583**

(43) Veröffentlichungstag der Anmeldung: **28.09.83 Patentblatt 83/39**

(45) Bekanntmachung des Hinweises auf die Patenterteilung: **17.09.86 Patentblatt 86/38**

(84) Benannte Vertragsstaaten: **AT BE CH DE FR GB IT LI LU NL SE**

(56) Entgegenhaltungen:
**CHEMICAL ABSTRACTS, Band 68, Nr. 5, 29. Januar 1968, Seite 1808, Nr. 18975k, Columbus, Ohio, USA L.D KOHN et al. "L- and meso-tartaric acid dehydrogenase (crystalline)"**
**CHEMICAL ABSTRACTS, Band 72, Nr. 7, 16. Februar 1970, Seite 69, Nr. 29049v, Columbus, Ohio, USA K.P. KLATT et al.: "Metabolism of tartaric acid by Penicillium charlesii"**
**CHEMICAL ABSTRACTS, Band 69, Nr. 1, 1. Juli 1968, Seite 33, Nr. 349v, Columbus, Ohio, USA L.D. KOHN et al.: "Tartaric acid metabolism. V. Crystalline tartrate dehydrogenase"**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH, Sandhofer Strasse 116, D-6800 Mannheim 31 (DE)**

(72) Erfinder: **Röder, Albert, Dr.rer.nat., Bahnhofstrasse 41, D-8124 Seeshaupt (DE)**
Erfinder: **Seidel, Hans, Dr.rer.nat., Waxensteinstrasse 6, D-8132 Tutzing (DE)**
Erfinder: **Giffhorn, Friedrich, Dr.rer.nat., Düstere Eichen Weg 38, D-3400 Göttingen (DE)**

**EP 0 089 640 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue L(+)-Tartrat-Dehydrogenase und ein Verfahren zu ihrer Herstellung sowie ein Verfahren und ein Reagens zur Bestimmung von L(+)-Tartrat und D(+)-Malat.

Von den drei möglichen Stereoisomeren der Weinsäure, die L(+)-, D(−)- und meso-Weinsäure, ist die L(+)-Weinsäure in der Natur weit verbreitet. Der Abbau von Weinsäure durch Mikroorganismen erfolgt auf unterschiedlichen Reaktionswegen. Ein Abbauweg führt über die enzymatische Oxidation mit Hilfe von Tartrat-Dehydratase. Dieses Enzym ist vor allem in Pseudomonaden nachgewiesen worden. Wegen seiner aussergewöhnlichen Instabilität konnte dieses Enzym noch nicht eingehend charakterisiert und auch nicht für einen quantitativen Nachweis von Weinsäure bzw. der Tartrat-Ionen verwendet werden.

Ein weiterer Weg des Weinsäure-Abbaus wird durch das Enzym Tartrat-Dehydrogenase eingeleitet. Primär entsteht bei dieser Reaktion das meta-stabile Produkt Oxaloglykolsäure. Auch die Tartrat-Dehydrogenase-Aktivität ist bisher in Pseudomonas-Arten nachgewiesen worden. Es ist prinzipiell möglich, sowohl mit Hilfe der Tartrat-Dehydratase als auch mit der Tartrat-Dehydrogenase eine enzymatische Bestimmung der Weinsäure auf der Basis der Nicotinamid-adenin-dinucleotid-(NAD) Absorption durchzuführen.

Es sind verschiedene Tartrat-Dehydrogenasen (Tartrate: NAD oxidoreductase, EC 1.1.1.93) bekannt, beispielsweise aus Pseudomonas-Arten und Penicillium charlesii. Diese Enzyme katalysieren die Umsetzung von Tartrat-Isomeren in Gegenwart von NAD als Coenzym zu Oxalglykolat. In J. Biol. Chem. 243 (1968) S. 2479–2485 wird beispielsweise eine Tartrat-Dehydrogenase aus Pseudomonas putida beschrieben, die sowohl L(+)-Tartrat als auch meso-Tartrat als auch meso-Tartrat in Gegenwart von NAD, Mangan(II)-Ionen und monovalente Kationen zu Oxalglykolat umzusetzen vermag. D(+)-Malat und L(-)-Malat werden von diesem Enzym nicht oxidiert.

Wegen der geringen spezifischen Aktivität, der grossen Instabilität der bisher bekannten Enzympräparationen und deren ungünstigen kinetischen Eigenschaften (hoher $K_m$-Wert für L(+)-Tartrat) blieben bisher alle Versuche erfolglos, eines der Enzyme für eine routinemässige, quantitative Bestimmung der L(+)-Weinsäure einzusetzen. Für den Nachweis von L(+)-Tartrat sind die bisher bekannten Tartrat-Dehydrogenasen auch deswegen nicht geeignet, weil sie neben der L-Form auch die ebenfalls natürlich vorkommende meso-Form abbauen.

Aufgabe der vorliegenden Erfindung war es daher, eine neue L(+)-Tartrat-Dehydrogenase bereit zu stellen, die hinreichend stabil ist, günstige kinetische Eigenschaften aufweist und von den möglichen Tartrat-Isomeren ganz spezifisch das L(+)-Tartrat-Isomere erfasst.

Überraschenderweise konnte nun gezeigt werden, dass aus Rhodopseudomonas-Arten, insbesondere Rhodopseudomonas sphaeroides Stämmen, die phototroph (Licht, anaerob) oder heterotroph (dunkel, aerob) auf L(+)-Tartrat gezüchtet worden sind, eine L(+)-Tartrat-Dehydrogenase isoliert werden kann, die hinreichend stabil ist, eine ausreichende spezifische Aktivität gegenüber L(+)-Tartrat aufweist, gute kinetische Eigenschaften besitzt und daher zum Nachweis von L(+)-Tartrat geeignet ist.

Die aus Rhodopseudomonas sphaeroides Stämmen isolierte L(+)-Tartrat-Dehdrogenase katalysiert auch die Umsetzung von D(+)-Malat zu Pyruvat und $CO_2$, d.h. sie weist neben der L(+)-Tartrat-Dehydrogenase-Aktivität auch eine D(+)-Malat-Dehdrogenase (decarboxylierend)-Aktivität auf. Das neue Enzym kann somit auch zum Nachweis von D(+)-Malat herangezogen werden.

Gegenstand der Erfindung ist eine neue L(+)-Tartrat-Dehydrogenase, erhältlich aus Rhodopseudomonas sphaeroides, die sowohl L(+)-Tartrat-Dehydrogenase-Aktivität als auch D(+)-Malat-Dehydrogenase (decarboxylierend)-Aktivität aufweist. Sowohl bei der Oxidation von L(+)-Tartrat als auch bei der Oxidation von D(+)-Malat benötigt das erfindungsgemässe Enyzm als spezifisches Cosubstrat NAD. Ferner ist die Anwesenheit von $Mn^{2+}$- oder $Mg^{2+}$-Ionen erforderlich. Der optimale pH-Wert für beide Aktivitäten liegt bei 7,5–9,0. Das Molekulargewicht des neuen Enzyms beträgt etwa 160 000. Das Enzym besteht aus vier Untereinheiten mit Molekulargewichten von jeweils 38 500. Der Isoelektrische Punkt liegt zwischen pH 5,0 und 5,2. Das Temperaturoptimum ist 50°C, die Aktivierungsenergien ($\Delta$ H°) betragen 54,4 kJ/mol für D(+)-Malat und 71,5 kJ/mol für L(+)-Tartrat.

Ein weiterer Gegenstand der Erfindung ist ein Verfahren zur Herstellung der neuen L(+)-Tartrat-Dehydrogenase. Zur Züchtung der erfindungsgemässen L(+)-Tartrat-Dehydrogenase können verschiedene Rhodopeudomonas sphaeroides Stämme eingesetzt werden. Besonders geeignet sind die Stämme Rhodopseudomonas sphaeroides DSM 158, DSM 159, DSM 160 und DSM 2303, die bei der Deutschen Sammelstelle von Mikroorganismen (DSM) hinterlegt sind.

Die Kultivierung der Rhodopseudomonas sphaeroides Stämme kann in üblicher Weise phototroph oder heterotroph durchgeführt werden. Das Wachstumsverhalten der Mikroorganismen zeigt nur geringe Abhängigkeit von diesen grundlegenden Kulturbedingungen.

Als Nährmedium wird ein Medium verwendet, das als wesentlichen Bestandteil L(+)-Weinsäure enthält. Ferner sind die in solchen Medien üblichen Stoffe, wie beispielsweise verschiedene anorganische Salze, Spurenelemente und Vitamine enthalten. Beide Aktivitäten der L(+)-Tartrat-Dehydrogenase (gegen L(+)-Tartrat und D(+)-Malat) werden durch L(+)-Tartrat parallel induziert. Nach dem Abbau des Induktors hört das Wachstum der Kultur und die Enzymbildung auf (vgl. Abb. 1). Die Aktivität der L(+)-Tartrat-Dehydrogenase bleibt auch während einer längeren stationären Phase erhalten, so dass proteolytische Prozesse ausgeschlossen werden können.

Die besten Wachstumsbedingungen werden erreicht, wenn das Kulturmedium L(+)-Tartrat in einer Anfangskonzentration von 20–50 mmol/l, vorzugsweise 40–50 mmol/l, enthält.

Durch Zusatz verschiedener Stoffe, z.B. mehrwertige Alkohole und Kohlenhydrate, zu dem Nährmedium, lässt sich die Wachstumsrate weiter erhöhen. So bewirkt beispielsweise ein Zusatz von Glycerin bzw. Glucose in einer Konzentration von jeweils 5–20 mmol/l, vorzugsweise 10 mmol/l, eine Steigerung der Aktivität der L(+)-Tartrat-Dehydrogenase.

Nach Abschluss der Kultivierung werden die Zellen in bekannter Weise geerntet. Die Zellextrakte weisen neben der L(+)-Tartrat-Dehydrogenase-Aktivität stets eine D(+)-Malat-Dehydrogenase(decarboxylierend)- sowie eine L(−)-Malat-Dehydrogenase- und Aldehyd-Dehydrogenase-Aktivität auf. Die rohen Zellextrakte können nach bekannten Methoden (Protaminsulfatfällung, Hitzeschritt, Ammoniumsulfatfällung, chromatographische Reinigung u.a.) weiter gereinigt werden.

Bei allen Reinigungsschritten bleibt das Verhältnis von D(+)-Malat-Dehydrogenase(decarboxylierend)- zur L(+)-Tartrat-Dehydrogenase-Aktivität konstant, während die L(−)-Malat-Dehydrogenase- und Aldehyd-Dehydrogenase-Aktivität quantitativ entfernt werden kann. Dies ist ein Hinweis darauf, dass die D(+)-Malat-Dehydrogenase(decarboxylierend)-Aktivität Bestandteil der L(+)-Tartrat-Dehydrogenase ist. Hierfür sprechen auch die folgenden Experimente bzw. Schlussfolgerungen:

1. Die spezifischen Aktivitäten von Zellen, die auf L(+)-Tartrat gewachsen waren, wurden verglichen mit der spezifischen Aktivität von Zellen, die auf Acetat, Succinat, Fumarat oder L(−)-Malat und auch D(+)-Malat gewachsen waren. Es hat sich gezeigt, dass sowohl die L(+)-Tartrat-Dehydrogenase- als auch D(+)-Malat-Dehydrogenase(decarboxylierend)-Aktivität von auf L(+)-Tartrat gewachsenen Zellen 40 bis 50fach höher liegt, als in Extrakten von Zellen, die auf Acetat, Succinat, Fumarat oder L(−)-Malat gewachsen waren, bzw. 5fach höher als in Extrakten von Zellen, die auf D(+)-Malat gewachsen waren. Es folgt daraus, dass L(+)-Tartrat beide Aktivitäten in gleicher Weise induziert und verglichen mit D(+)-Malat der bessere Induktor darstellt.

2. Beide Aktivitäten haben dasselbe pH-Optimum. Sie benötigen beide diesselben Effektoren und Cosubstrate.

3. Die Gelfiltration an Sepharose® 6B führt zur Elution beider Aktivitäten innerhalb eines symmetrischen Peaks.

4. Beide Aktivitäten werden bei 45, 50 und 55°C mit derselben Rate inaktiviert.

5. Durch Aktivitätsfärbung mit Phenazinmethosulfat und p-Nitroblautetrazoliumchlorid in Polyacrylamid-Gelen konnte dieselbe Bande als L(+)-Tartrat-aktiv sowie D(+)-Malat-aktiv nachgewiesen werden.

Im folgenden sind für die erfindungsgemässe L(+)-Tartrat-Dehydrogenase (TDH) aus verschiedenen Rhodopseudomonas sphaeroides Stämmen die spezifischen Aktivitäten der TDH (U/mg) gegenüber L(+)-Tartrat im Rohextrakt und nach dem Hitzeschritt angegeben:

| Stamm | Spezifische Aktivität (U/mg) gegen L(+)-Tartrat | |
| --- | --- | --- |
| | im Rohextrakt | nach dem Hitzeschritt |
| DSM 158 | 0,006 | 0,014 |
| DSM 159 | 0,01 | 0,022 |
| DSM 160 | 0,017 | 0,028 |
| DSM 2303 | 0,02 | 0,032 |

Die erfindungsgemässe L(+)-Tartrat-Dehydrogenase (TDH) vermag mit guter Reaktivität und Selektivität die beiden folgenden enzymatischen Reaktionen zu katalysieren:

$$(1)\quad L(+)\text{-Tartrat} + NAD^{\oplus} \xrightarrow{TDH} \text{Oxalglycolat} + NADH + H^{\oplus}$$

$$(2)\quad D(+)\text{-Malat} + NAD^{\oplus} \xrightarrow{TDH} \text{Pyruvat} + CO_2 + NADH + H^{\oplus}$$

Bei beiden enzymatischen Reaktionen liegt das pH-Optimum bei 7,5–9,0. Für beide Umsetzungen werden Nicotinamid-adenin-dinucleotid (NAD) als Cosubstrat und $Mn^{2+}$- oder $Mg^{2+}$-Ionen als essentielle Kationen benötigt. Folgende $K_m$-Werte wurden in bekannter Weise gemessen (30°C; 0,1 mol/l Tris-HCl-Puffer, Ph 8,5; 0,4 mmol/l $MnCl_2$; 1 mmol/l Mercaptoethanol; 50 mmol/l Ammoniumsulfat; 20 mmol/l KCl; 0,12 U L(+)-Tartrat-Dehydrogenase; die $K_m$-Werte für $NAD^+$ bzw. $Mn^{2+}$ wurden jeweils bei sättigender Konzentration der anderen Reaktionsteilnehmer bestimmt):

| | |
| --- | --- |
| L(+)-Tartrat | $2{,}3 \times 10^{-3}$ mol/l |
| $NAD^+$ | $2{,}8 \times 10^{-4}$ mol/l |
| $Mn^{2+}$ | $1{,}6 \times 10^{-5}$ mol/l |
| D(+)-Malat | $1{,}7 \times 10^{-4}$ mol/l |
| $NAD^+$ | $1{,}3 \times 10^{-4}$ mol/l |
| $Mn^{2+}$ | $1{,}6 \times 10^{-5}$ mol/l |

Die erfindungsgemässe L(+)-Tartrat-Dehydrogenase ist weitgehend stabil. Das Enzym verliert in lyophilisierter Form bzw. als Lösung bei 25°C innerhalb von 30 Tagen nur geringfügig an Aktivität. Die L(+)-Tartrat-Dehydrogenase wird bevorzugt in Form einer Lösung in einer Ammoniumsulfat-Lösung (100 mmol/l) verwendet. Eine gesonderte Zugabe von Ammoniumionen zu den im folgenden beschriebenen Testzusammensetzungen erübrigt sich damit.

Die Stabilität der L(+)-Tartrat-Dehydrogenase, ihre hohe Spezifität und ihre hohe Affinität zu L(+)-Tartrat machen das erfindungsgemässe Enzym für die Bestimmung von L(+)-Tartrat gemäss dem oben angegebenen Reaktionsschema (1) geeignet.

Gegenstand der Erfindung ist daher ferner ein Verfahren und ein Reagens zur Bestimmung von L(+)-Tartrat in Flüssigkeiten, das dadurch gekennzeichnet ist, dass L(+)-Tartrat in Gegenwart von NAD und $Mn^{2+}$- oder $Mg^{2+}$-Ionen mit Hilfe von L(+)-Tartrat-Dehydrogenase in Oxalglykolat unter Bildung von NADH übergeführt und das entstehende NADH in bekannter Weise bestimmt wird.

Die Bestimmung des entstehenden NADH kann beispielsweise photometrisch erfolgen, indem die Zunahme der Extinktion bei 365 nm (Hg) gemessen wird. Hierzu wird die Tartrathaltige Probelösung zweckmässigerweise mit einem Puffersystem, pH 7,5–9,0, beispielsweise einem Tris-HCl-Puffer, pH 8,5, versetzt. Es werden NAD und $Mn^{2+}$- oder $Mg^{2+}$-Ionen sowie gegebenenfalls weitere Hilfsstoffe, wie Detergentien und/oder Stabilisierungsmittel zugegeben. Die enzymatische Reaktion wird mit erfindungsgemässer L(+)-Tartrat-Dehydrogenase gestartet. Die Messung wird vorteilhafterweise bei Raumtemperatur oder leicht erhöhter Temperatur durchgeführt.

Bei Raumtemperatur liegt die Reaktionsdauer zwischen 25 und 60 Min. Bei einem Einsatz von 0,1–3,0 U Tartrat-Dehydrogenase pro Test werden dabei 3–50% Tartrat wiedergefunden. Wird die Reaktionstemperatur auf 30–40°C erhöht, so wird bei 3 U Tartrat-Dehydrogenase pro Test eine 50%-ige Tartrat-Umsetzung innerhalb von 20 Min. erreicht.

Die für die Nachweisreaktion erforderlichen Stoffe werden zweckmässig in folgenden Konzentrationen verwendet:

0,01–1,00 mol/l Puffer, pH 7,5–9,0
0,1–15 mmol/l Mangan(II)- bzw. Magnesium-Salz
2–50 mmol/l NAD
0,01–5 U/ml erfindungsgemässe L(+)-Tartrat-Dehydrogenase in wässriger Ammoniumsulfat-Lösung (100 mmol/l).

Besonders bevorzugt ist folgende Testzusammensetzung:

20–50 mmol/l Tris-HCl-Puffer, pH 8,5
4–10 mmol/l Mangan(II)-chlorid
15–25 mmol/l NAD
0,1–2 U/ml erfindungsgemässe L(+)-Tartrat-Dehydrogenase in wässriger Ammoniumsulfat-Lösung (100 mmol/l).

Vorteilhafter und schneller ist eine Tartrat-Umsetzung zu erreichen, wenn Reaktion (1) mit folgender Reaktion (3) gekoppelt wird:

$$(3)\quad NADH + Tetrazoliumsalz + H^{\oplus} \longrightarrow NAD^{\oplus} + Formazan$$

Hierzu werden der Testlösung zusätzlich ein Tetrazoliumsalz, ein geeigneter Katalysator sowie gegebenenfalls weitere übliche Hilfsstoffe zugesetzt. Beispielsweise kann die Testreaktion mit Nitrotetrazolium-blau (NTB) als Tetrazoliumsalz in Gegenwart von Kaliumchlorid und Ammoniumchlorid durchgeführt werden.

Die Reaktion läuft in etwa 30 Min. ab, wobei ca. 25–30% Tartrat wiedergefunden werden. Bei Einsatz von 2,5-Diphenyl-3[4,5-dimethyl-thiazolyl-(2)]-tetrazoliumbromid (MTT) als Tetrazoliumsalz wird die Reaktionsgeschwindigkeit deutlich erhöht. Innerhalb von 10 Min. werden 50% Tartrat umgesetzt.

Eine vorteilhafte Ausführungsform der erfindungsgemässen L(+)-Tartrat-Bestimmung ist, den enzymatischen Tartrat-Abbau (1) mit einer Tetrazoliumsalz-Reaktion (3) zu koppeln, wobei als Tetrazoliumsalz 2-p-Jodphenyl-3-p-nitrophenyl-5-phenyl-tetrazoliumchlorid (INT) eingesetzt und ausserdem Diaphorase und Polyoxyethylenalkylarylether (Triton® X 100) zugesetzt werden. Diese Bestimmungsvariante wird vorteilhaft mit folgenden Testkonzentrationen durchgeführt:

0,01–1,00 mol/l Puffer, pH 7,5–9,0
1–100 mmol/l Alkalichlorid
0,1–100 mmol/l Mangan(II)- bzw. Magnesium-Salz
1–100 mmol/l NAD
0,05–1,0% (v/v) Polyoxyethylenalkylarylether (Triton® X 100)
0,1–5,0 U/ml Diaphorase
0,01–1,0 mmol/l Tetrazoliumsalz
0,01–10 U/ml erfindungsgemässe L(+)-Tartrat-Dehydrogenase in wässriger Ammoniumsulfat-Lösung (100 mmol/l).

Besonders bevorzugt ist folgende Testzusammensetzung:

65–80 mmol/l Tris-HCl-Puffer, pH 8,5
10–20 mmol/l Kaliumchlorid
0,3–0,6 mmol/l Mangan(II)-chlorid
4–10 mmol/l NAD
0,1–0,2% (v/v) Polyoxyethylenalkylarylether (Triton® X 100)
0,5–1,0 U/ml Diaphorase
0,05–0,2 mmol/l INT
0,15–0,25 U/ml erfindungsgemässe L(+)-Tartrat-Dehydrogenase in wässriger Ammoniumsulfat-Lösung (100 mmol/l).

Die erfindungsgemässe L(+)-Tartrat-Dehydrogenase oxidiert auch D(+)-Malat in Gegenwart von NAD und Mangan(II)-Ionen zu Pyruvat und $CO_2$. Das dabei entstehende NADH kann ebenfalls nach bekannten Methoden bestimmt werden, z.B. direkt photometrisch bei 365 nm (Hg) oder aber auch nach Zusatz eines Tetrazoliumsalzes und gegebenenfalls weiterer Zusatzstoffe über den sich bildenden Formazan-Farbstoff.

Gegenstand der vorliegenden Erfindung ist somit ferner ein Verfahren und ein Reagens zur Bestimmung von D(+)-Malat in Lösungen, insbesondere in Fruchtsäften, das dadurch gekennzeichnet ist, dass D(+)-Malat in Gegenwart von NAD und $Mn^{2+}$- oder $Mg^{2+}$-Ionen zu Pyruvat und $CO_2$ unter Bildung von NADH oxidiert und das entstehende NADH nach bekannten Methoden gemessen wird.

Das Bestimmungsverfahren wird bei konstanter Messtemperatur durchgeführt, die zwischen 20 und 40°C, vorzugsweise zwischen 25 und 30°C liegt. Zur Bestimmung von D(+)-Malat wird die D(+)-Malat-haltige Probe mit einer Lösung in Kontakt gebracht, die in einem Puffersystem mit einem Pufferbereich zwischen pH 7,5 und 9,0, vorzugsweise Tris-Puffer, pH 8,5 NAD und Mangan(II)-chlorid sowie gegebenenfalls weitere Zusatzstoffe, wie beispielsweise Detergentien und/oder Stabilisierungsmittel, enthält, in Kontakt gebracht. Durch Zugabe von L(+)-Tartrat-Dehydrogenase wird die enzymatische Reaktion gestartet. Aus den Extinktionen vor Zusatz des Enzyms und nach Beendigung der enzymatischen Reaktion wird die Extinktionsdifferenz ermittelt, aus der die Anfangskonzentration an D(+)-Malat in der Probe ermittelt werden kann. Um die Absorption der eingesetzten Reagentien berücksichtigen zu können, wird parallel zur Probe eine Leerwert-Messung durchgeführt, wobei anstelle der Probe eine entsprechende Menge an Wasser dem Testansatz zugefügt wird.

Die für die Bestimmung erforderlichen Substanzen werden vorteilhafterweise in folgenden Konzentrationen eingesetzt:

0,01–1 mol/l Puffer, pH 7,5–9,0
0,1-100 mmol/l Mangan(II)- bzw. Magnesium-Salz
1–100 mmol/l NAD
0,01–1 U/ml erfindungsgemässe L(+)-Tartrat-Dehydrogenase in wässriger Ammoniumsulfat-Lösung (100 mmol/l).

Folgende Testzusammensetzung ist besonders bevorzugt:

0,05–0,2 mol/l Tris-HCl-Puffer, pH 8,5
3–10 mmol/l Mangan(II)-chlorid
5–20 mmol/l NAD
0,01–0,1 U/ml erfindungsgemässe L(+)-Tartrat-Dehydrogenase in wässriger Ammoniumsulfat-Lösung (100 mmol/l).

Die Konzentration an D(+)-Malat in der Testlösung sollte den Wert von 0,25 mmol/l nicht übersteigen. Sind in der zu bestimmenden Probe höhere Konzentrationen an D(+)-Malat zu erwarten, so kann durch entsprechende Verdünnung der vorteilhafte Konzentrationsbereich von unter 0,25 mmol/l eingestellt werden.

Der Nachweis von D(+)-Malat mit der erfindungsgemässen L(+)-Tartrat-Dehydrogenase ist sehr spezifisch. L(−)-Äpfelsäure bzw. L(−)-Malat reagiert nicht.

Die Bestimmung von D(+)-Malat in Fruchtsäften ist vor allen Dingen deswegen von Bedeutung, weil D(+)-Äpfelsäure oft in unzulässiger Weise den Fruchtsäften zugesetzt wird. Fruchtsäfte enthalten üblicherweise keine D(+)-Äpfelsäure. Eine positive Nachweisreaktion mit L(+)-Tartrat-Dehydrogenase deutet folglich auf eine Verfälschung des Fruchtsaftes durch Zusatz von D(+)-Äpfelsäure hin.

Im Gegensatz zu dem oben beschriebenen Tartratabbau mit Hilfe der L(+)-Tartrat-Dehydrogenase, der jeweils nur bis zu 30–50% erreicht wird, ist der D(+)-Malatabbau mit Hilfe der L(+)-Tartrat-Dehydrogenase vollständig. Zugesetzte D(+)-Äpfelsäure zu einer Lösung wird quantitativ wiedergefunden.

Die Erfindung wird durch die folgenden Beispiele näher erläutert:

Beispiel 1

L(+)-Tartrat-Dehydrogenase aus Rhodopseudomonas sphaeroides DSM 160

Anzucht der Zellen

Die Zellen von Rhodopseudomonas sphaeroides DSM 160 werden anaerob im Licht 36 Std. auf einem Kulturmedium angezogen, das folgende Bestandteile enthält:

3,0 g L(+)-Weinsäure
1,0 g $KH_2PO_4$
0,4 g $MgSO_4 \times 7H_2O$
0,4 g NaCl
0,5 g $NH_4Cl$
0,05 g $CaCl_2 \times 2H_2O$
10 ml Spurenelementlösung SL 4 nach N. Pfennig u.a., Arch. Mikrobiol. 55 (1966) S. 245-256
0,5 g Hefeextrakt
ad 1 l $H_2O$
pH 6,7 mit einer Mischung aus je 5 M KOH oder NaOH eingestellt.

Kultivierung in aerober Schüttelkultur

Jeweils 300 ml der in vorstehender Weise gewonnenen Zellsuspension werden in 1 l Erlenmeyer-Langhalskolben mit Schikanen bei 30°C auf dem Schütteltisch bei 150 Upm unter Zusatz des oben genannten Kulturmediums inkubiert.

In Abb. 1 ist der Verlauf des Tartratabbaus (-△-△-), der Absorption bei 650 nm (-□-□-) und der spezifischen Aktivität der TDH gegenüber L(+)-Tartrat (-O-O-) und D(+)-Malat (-O-O-) aufgetragen.

Kultivierung im Fermenter

Die Kultivierungsversuche im Schüttelkolben lassen sich ohne weiteres auf Fermenteransätze übertragen. 10 l Fermenter (Braun-Biostat) werden mit einem Kulturmedium gefüllt, das 10,5 g/l L(+)-Weinsäure (70 mmol/l) und 8 g/l Hefeextrakt enthält. Während der Kultivierung wird mit Luft 5 l/Min. belüftet. Die Temperatur wird bei 30°C gehalten. Die Rührerdrehzahl beträgt 400 Upm. Der pH-Wert wird während der Dauer der Kultivierung durch Zusatz entsprechender Mengen Salzsäure oder Phosphorsäure konstant bei pH = 6,7 gehalten.

Fermenter I wird mit 10% einer aeroben Vorkultur beimpft. Der pH-Wert wird mit 1N Salzsäure konstant gehalten. Fermenter II wird mit 10% einer aeroben Vorkultur beimpft. Der pH-Wert wird mit 5M Phosphorsäure (Verbrauch ca. 140 ml) konstant gehalten.

Fermenter III wird mit 20% Rückstand aus Fermenter II beimpft. Der pH-Wert wird wie bei Fermenter II mit 5M Phosphorsäure konstant gehalten. Nach 12 Std. wurde komplettes Kulturmedium nachgefüllt.

Wie bei der Kultur im Schüttelkolben werden nach 24 Std. die erwähnten Parameter bestimmt. Unter der berechtigten Annahme, dass $A_{650}=1$ ungefähr 6 U TDH/l entspricht, ergeben sich bei den einzelnen Fermenter-Ansätzen folgende Enzymausbeuten:

| Fermenter | TDH |
|---|---|
| I | 42 |
| II | 54 |
| III | 90 |

Anreicherung der L(+)-Tartrat-Dehydrogenase

Die bei der Kultivierung erhaltene Zellmasse wird in einer Pufferlösung (3 ml Puffer/g Zellmasse) suspendiert, die 100 mmol/l Tris-HCl-Puffer, pH 7,0, 0,4 mmol/l Mangan(II)-chlorid und 5 mmol/l Mercaptoethanol enthält. Die Zellen werden mit der Frenchpresse bei einem Druck zwischen 96,105 mPa und 137,293 mPa aufgeschlossen. Zelltrümmer werden anschliessend durch Zentrifugation (30 Min. bei 20 000 g) abgetrennt.

Dem erhaltenen Zellextrakt wird Protaminsulfat-Lösung tropfenweise zugesetzt. Der ausgefallene Niederschlag wird wie oben angegeben abzentrifugiert.

Der Überstand wird 10 Min. auf 53°C erhitzt. Die Proteinlösung wird anschliessend in Eis abgekühlt und wie oben angegeben zentrifugiert.

Aus dem erhaltenen Überstand wird das Enzym mit gesättigter Ammoniumsulfat-Lösung, die mit 5 mmol/l Mercaptoethanol versetzt ist, ausgefällt.

Der Niederschlag wird in Dialyse-Puffer suspendiert, der 50 mmol/l Tris-HCl-Puffer, pH 7,0, 0,4 mmol/l Mangan(II)-chlorid und 5 mmol/l Mercaptoethanol enthält. Es wird 12–15 Stunden gegen das 50fache Volumen des Dialysepuffers dialysiert. Eine zurückbleibende Trübung wird abzentrifugiert.

Eine weitere Aufreinigung kann durch Chromatographie an DEAE-Cellulose und/oder an einem DEAE-Sephadex® A50-Ionenaustauscher erfolgen.

In der folgenden Tabelle sind Proteinausbeuten, die Aktivitäten gegenüber L(+)-Tartrat, D(+)-Malat und L(−)-Malat sowie die spezifische Aktivität gegenüber L(+)-Tartrat nach den verschiedenen Reinigungsoperationen zusammengestellt:

| Anreicherungsschritt | Gesamtprotein (mg) | Gesamtaktivität (U) | | | Spez. Aktivität (U/mg) | Faktor |
|---|---|---|---|---|---|---|
| | | L(+)-Tartrat | D(+)-Malat | L(−)-Malat | L(+)-Tartrat-DH | |
| Rohextrakt | 1234 | 20,8 | 228 | 2004 | 0,017 | 1 |
| Protaminsulfatfällung | 1134 | 15,2 | 209 | 1980 | | |
| Hitzeschritt | 362 | 19,5 | 218 | 27 | 0,054 | 3,2 |
| Ammoniumsulfatfällung | 199 | 19,0 | 215 | 11 | 0,095 | 5,6 |
| DEAE-Cellulose | 50 | 14,0 | 161 | - | 0,28 | 16 |
| DEAE-Sephadex® A50 | 11 | 13,5 | 155 | - | 1,23 | 72 |

Mit dem angereicherten Enzym wird die Spezifität der L(+)-Tartrat-Dehydrogenase in bekannter Weise bestimmt. Hierzu wird beispielsweise zu der Enzym-haltigen Lösung Tris-HCl-Puffer, pH 8,5, und eine Ammonium-, Kalium- und Mangan(II)-Ionen enthaltende NAD-Lösung gegeben. Die Reaktion wird durch Zugabe der in der folgenden Tabelle angegebenen Substrate in einer Konzentration von 10 µmol/ml gestartet. Die Testansätze enthielten jeweils 20 µl Enzym, mit Ausnahme bei D(+)-Malat als Substrat. In diesem Falle wurden nur 2 µl Enzym eingesetzt. Die Messergebnisse sind in der folgenden Tabelle aufgeführt.

| Substrat | △ E/Min. |
|---|---|
| L(+)-Tartrat | 0,47 |
| D(−)-Tartrat | 0 |
| meso-Tartrat | 0 |
| L(−)-Malat | 0 |
| D(+)-Malat | 0,43 |
| Laktat | 0 |
| Hydroxypyruvat | 0 |
| Glycolat | 0 |
| Glycerat | 0 |
| Succinat | 0 |
| Isocitrat | 0 |
| Citrat | 0 |
| $NADP^+$ + L(+)-Tartrat | 0 |
| $NADP^+$ + D(+)-Malat | 0 |

Beispiel 2

L(+)-Tartrat-Dehydrogenase aus Rhodopseudomonas sphaeroides DSM 2303

Rhodopseudomonas sphaeroides DSM 2303 wurde, wie in Beispiel 1 beschrieben, vorkultiviert, im Fermenter gezüchtet und in üblicher Weise geerntet. Danach wurde die rohe L(+)-Tartrat-Dehydrogenase den bekannten Reinigungsoperationen, wie Hitzeschritt, Ammoniumsulfat-Fraktionierung, chromatographische Trennung über DEAE-Cellulose und DEAE-Sephadex® A 50 unterworfen. Man erhält ein Enzym mit einer spezifischen Aktivität gegenüber L(+)-Tartrat von 400 U/g Protein.

Im Rohextrat noch vorhandene Fremdaktivitäten, wie L(−)-Malat-Dehydrogenase- und Aldehyd-Dehydrogenase-Aktivität, konnten bei der Aufreinigung des Enzyms beseitigt werden. In der folgenden Tabelle ist die Abreichung an diesen Fremdaktivitäten nach einzelnen Reinigungsschritten aufgezeigt.

| Reinigungsschritt | Gesamtaktivität (U) L(−)-Malat-Dehydrogenase | Aldehyd-Dehydrogenase |
|---|---|---|
| Zellextrakt | 1270 | |
| Ammoniumsulfatfällung | 780 | 9,3 |
| DEAE-Cellulose | 63 | 1,2 |
| DEAE-Sephadex® A50 | 0 | 0 |

Beispiel 3

Photometrische Bestimmung von L(+)-Tartrat

In Kunststoff-Küvetten (Schichtdicke: 1 cm, Testvolumen: 1,32 ml) werden folgende Lösungen pipettiert:

| | Leerwert (ml) | Probe (ml) | Konzentration im Test |
|---|---|---|---|
| Tris, pH 8,5, 50 mmol/l | 1,00 | 1,00 | 38 mmol/l |
| Manganchlorid, 70 mmol/l | 0,10 | 0,10 | 5,3 mmol/l |
| NAD, 230 mmol/l | 0,10 | 0,10 | 17,1 mmol/l |
| Probelösung (Weinsäure) | - | 0,10 | bis ca. 250 µmol/l |
| Wasser | 0,10 | - | - |

Der Inhalt sowohl der Probe- als auch der Leerwert-Küvette wird vermischt. Danach wird die Ausgangsextinktion $E_1$ bei der Wellenlänge 365 nm (Hg) gemessen (Messtemperatur: 37°C bzw. Raumtemperatur; Messung gegen Luft). Zum Starten der enzymatischen Umsetzung werden beide Küvetten mit 0,1–3,0 U (≙ 0,03–1 U/ml Testlösung) der erfindungsgemässen L(+)-Tartrat-Dehydrogenase versetzt. Es wird erneut durchmischt und bei 37°C bzw. Raumtemperatur bis zum Reaktionsstillstand inkubiert. Danach wird der Extinktionswert $E_2$ gemessen. Aus den Extinktionsdifferenzen $\Delta E = E_2\text{-}E_1$ wird mit Hilfe der Gleichung $C = \Delta E \cdot 0{,}5827$ [g/l] die Ausgangs-Tartrat-Konzentration in der Probelösung bestimmt.

Beispiel 4

Tartrat-Bestimmung über Formazan-Bildung

Halbmikro-Küvetten (Schichtdicke: 1 cm, Testvolumen: 1,475 ml) werden mit folgenden Lösungen gefüllt:

| | Leerwert (ml) | Probe (ml) | Konzentration im Test |
|---|---|---|---|
| Tris, pH 8,5, 100 mmol/l | 1,000 | 1,000 | 68 mmol/l |
| KCl, 220 mmol/l | 0,100 | 0,100 | 15 mmol/l |
| $MnCl_2$, 120 mmol/l | 0,005 | 0,005 | 0,41 mmol/l |
| NAD, 75 mmol/l | 0,100 | 0,100 | 5,1 mmol/l |
| Triton® X 100, 10% | 0,020 | 0,020 | 0,13% |
| Diaphorase, 16 U/ml | 0,050 | 0,050 | 0,54 U/ml |
| INT, 1,19 mmol/l | 0,100 | 0,100 | 0,08 mmol/l |
| Probe (Weinsäure) | - | 0,100 | bis 40 µmol/l |
| Wasser | 0,100 | - | - |

Der Inhalt der Probe- und der Leerwert-Küvette wird vermischt. Die Ausgangsextinktion $E_1$ wird bei der Wellenlänge 492 nm (Hg) gemessen (Messtemperatur 37°C; Messung gegen Luft). Zum Starten der enzymatischen Reaktion werden beide Küvetten mit 0,01 ml einer erfindungsgemässen L(+)-Tartrat-Dehydrogenase-Präparation, die eine Aktivität von 46 U/ml aufweist, versetzt (Endkonzentration im Testansatz: 0,36 U/ml). Es wird erneut durchmischt und bis zum Reaktionsstillstand bei 37°C inkubiert. Danach wird die Extinktion $E_2$ gemessen.

Die enzymatische Reaktion ist nach ca. 20 Min. vollständig abgelaufen, ohne dass irgendwelche Schleichreaktionen stattfinden. Die Extinktionsdifferenz ΔE liegt bei ungefähr 0,8. Aus der Extinktionsdifferenz ΔE für eine Probe mit unbekanntem Tartrat-Gehalt lässt sich durch Vergleich mit Standardwerten, die durch Messen bekannter Tartrat-Konzentrationen ermittelt werden können, die Ausgangs-Tartrat-Konzentration in der Probe ermitteln.

Beispiel 5
Bestimmung von D(+)-Malat in Fruchtsäften

Küvetten (Schichtdicke: 1 cm; Testvolumen: 2,96 ml; Mess-Wellenlänge: 365 nm (Hg)) werden bei 25°C mit den folgenden Lösungen gefüllt:

| Ausgangslösung | Probe (ml) | Leerwert (ml) | Konzentrationen im Test |
|---|---|---|---|
| Tris-Puffer, pH 8,5; 0,1 mol/l | 2,70 | 2,70 | 0,09 mol/l |
| Manganchlorid, 0,19 mol/l | 0,01 | 0,01 | 0,6 mmol/l |
| NAD, 0,15 mol/l | 0,10 | 0,10 | 5,1 mmol/l |
| Probelösung (ca. 1 mg D-Äpfelsäure/ml) | 0,10 | - | bis zu 0,25 mmol/l |
| $H_2O$ | - | 0,10 | |

Der Inhalt beider Küvetten wird durchmischt und nach ca. 2 Min. wird die Extinktion $E_1$ gemessen. Danach wird durch Zusatz von 0,05 ml erfindungsgemässer Tartrat-Dehydrogenase mit einer Aktivität von 1,8 U/ml (Konzentration im Testvolumen: 30 mU/ml) die enzymatische Reaktion gestartet. Nachdem die Extinktion nicht mehr merklich zunimmt (nach 5-10 Min.), wird die Extinktion $E_2$ für Probe- und Leerwert gemessen. Aus den Extinktionsdifferenzen $E_2 - E_1$ für Probe und Leerwert wird der Wert ΔE berechnet, der Gehalt an D(+)-Äpfelsäure bzw. D(+)-Malat wird nach der Formel: D(+)-Äpfelsäure =ΔE × 1,167 [g/l Probelösung] berechnet. Gemäss den oben stehenden Testbedingungen wird die D-Äpfelsäure zu 99% wiedergefunden.

**Patentansprüche**

1. L(+)-Tartrat-Dehydrogenase, erhältlich aus Rhodopseudomonas sphaeroides, dadurch gekennzeichnet, dass sie L(+)-Tartrat-Dehydrogenase- und D(+)-Malat-Dehydrogenase (decarboxylierend)-Aktivität aufweist.

2. L(+)-Tartrat-Dehydrogenase gemäss Anspruch 1, dadurch gekennzeichnet, dass sie folgende zusätzliche Eigenschaften aufweist:
a) als Cosubstrat wird NAD benötigt;
b) $Mn^{2+}$- oder $Mg^{2+}$-Ionen sind essentielle Kationen;
c) optimaler pH-Wert von 7,5-9,0;
d) optimale Temperatur von 50°C;
e) Molekulargewicht von etwa 160 000;
f) Michaelis-Konstanten: L(+)-Tartrat $2.3 \times 10^{-3}$ mol/l
D(+)-Malat $1.7 \times 10^{-4}$ mol/l;
g) isoelektrischer Punkt bei pH 5,0-5,2.

3. Verfahren zur Herstellung von L(+)-Tartrat-Dehydrogenase, dadurch gekennzeichnet, dass man Rhodopseudomonas sphaeroides in einem üblichen Nährmedium, das als Kohlenstoffquelle L(+)-Tartrat enthält, kultiviert und aus der resultierenden Kulturbrühe die L(+)-Tartrat-Dehydrogenase in üblicher Weise gewinnt.

4. Verfahren gemäss Anspruch 3, dadurch gekennzeichnet, dass das Nährmedium L(+)-Tartrat in einer Anfangskonzentration von 20-50 mmol/l enthält.

5. Verfahren gemäss einem der Ansprüche 3 und 4, dadurch gekennzeichnet, dass als Mikroorganismen-Stamm Rhodopseudomonas sphaeroides DSM 158, Rhodopseudomonas sphaeroides DSM 159, Rhodopseudomonas sphaeroides DSM 160 oder Rhodopseudomonas sphaeroides DSM 2303 verwendet wird.

6. Verfahren zur Bestimmung von L(+)-Tartrat in Lösungen, dadurch gekennzeichnet, dass L(+)-Tartrat in Gegenwart von NAD und $Mn^{2+}$- oder $Mg^{2+}$-Ionen mit Hilfe von L(+)-Tartrat-Dehydrogenase gemäss einem der Ansprüche 1 oder 2 zu Oxalglykolat unter Bildung von NADH umgesetzt wird und das gebildete NADH nach bekannten Methoden bestimmt wird.

7. Verfahren gemäss Anspruch 6, dadurch gekennzeichnet, dass ein Tetrazoliumsalz, ein geeigneter Katalysator und weitere übliche Hilfsstoffe zugesetzt werden und das entstehende Formazan photometrisch bestimmt wird.

8. Reagens zur Bestimmung von L(+)-Tartrat gemäss Anspruch 6, enthaltend
0,01–1,00 mol/l Puffer, pH 7,5–9,0
0,1–15 mmol/l Mangan(II)- bzw. Magnesium-Salz
2–50 mmol/l NAD
0,01–5 U/ml L(+)-Tartrat-Dehydrogenase gemäss einem der Ansprüche 1 oder 2.

9. Reagens zur Bestimmung von L(+)-Tartrat gemäss Anspruch 7, enthaltend
0,01–1,00 mol/l Puffer, pH 7,5–9,0
1–100 mmol/l Alkalichlorid
0,1–100 mmol/l Mangan(II)- bzw. Magnesium-Salz
1–100 mmol/l NAD
0,05–1,0% (v/v) Polyoxyethylenalkylarylether
0,1–5 U/ml Diaphorase
0,01–1,0 mmol/l Tetrazoliumsalz
0,01–10 U/ml L(+)-Tartrat-Dehydrogenase gemäss einem der Ansprüche 1 oder 2.

10. Verfahren zur Bestimmung von D(+)-Malat in Lösungen, dadurch gekennzeichnet, dass man D(+)-Malat in Gegenwart von NAD und $Mn^{2+}$- oder $Mg^{2+}$-Ionen mit Hilfe von L(+)-Tartrat-Dehydrogenase gemäss einem der Ansprüche 1 oder 2 Pyruvat und $CO_2$ unter Bildung von NADH überführt und das entstehende NADH in üblicher Weise bestimmt.

11. Reagens zur Bestimmung von D(+)-Malat gemäss Anspruch 10, enthaltend
0,01–1 mol/l Puffer, pH 7,5–9,0
0,1–100 mmol/l Mangan(II)- bzw. Magnesium-Salz
1–100 mmol/l NAD
0,01–1 U/ml L(+)-Tartrat-Dehydrogenase gemäss einem der Ansprüche 1 oder 2.

## Claims

1. L(+)-Tartrate dehydrogenase obtainable from Rhodopseudomonas sphaeroides characterised in that it displays L(+)-tartrate dehydrogenase and D(+)-malate dehydrogenase (decarboxylating) activity.

2. L(+)-Tartrate dehydrogenase according to claim 1 or 2, characterised in that it displays the following additional properties:
a) requires NAD as co-substrate;
b) $Mn^{2+}$ or $Mg^{2+}$ ions are essential cations;
c) optimum pH value of 7.5–9.0;
d) optimum temperature of 50°C.;
e) molecular weight of about 160,000;
f) Michaelis constants: L(+)-tartrate $2.3 \times 10^{-3}$ mol/l.
D(+)-malate $1.7 \times 10^{-4}$ mol/l;
g) isoelectric point at 5.0–5.2.

3. Process for the production of L(+)-tartrate dehydrogenase, characterised in that one cultures Rhodopseudomonas sphaeroides in a conventional nutrient medium which contains L(+)-tartrate as carbon source and obtains the L(+)-tartrate dehydrogenase in the usual way from the resultant culture broth.

4. Process according to claim 3, characterised in that the nutrient medium contains L(+)-tartrate in an initial concentration of 20–50 mmol/l.

5. Process according to one of claims 3 and 4, characterised in that, as micro-organism strain, there is used Rhodopseudomonas sphaeroides DSM 158, Rhodopseudomonas sphaeroides DSM 159, Rhodopseudomonas sphaeroides DSM 160 or Rhodopseudomonas sphaeroides DSM 2303.

6. Process for the determination of L(+)-tartrate in solutions, characterised in that L(+)-tartrate is reacted in the presence of NAD and $Mn^{2+}$ or $Mg^{2+}$ ions with the help of L(+)-tartrate dehydrogenase according to one of claims 1 or 2 to give oxaloglycolate with the formation of NADH and the NADH formed is determined according to known methods.

7. Process according to claim 6, characterised in that a tetrazolium salt, a suitable catalyst and further conventional adjuvants are added and the resultant formazan is determined photometrically.

8. Reagent for the determination of L(+)-tartrate according to claim 6, containing
0.01–1.00 mol/l. buffer, pH 7.5–9.0
0.1–15 mmol/l. manganese (II) or magnesium salt
2–50 mmol/l. NAD
0.01–5 U/ml. L(+)-tartrate dehydrogenase according to one of claims 1 or 2.

9. Reagent for the determination of L(+)-tartrate according to claim 7, containing
0.01–1.00 mol/l. buffer, pH 7.5–9.0
1–100 mmol/l. alkali metal chloride
0.1–100 mmol/l. manganese (II) or magnesium salt
1–100 mmol/l. NAD
0.05–1.0% (v/v) polyoxyethylene alkyl aryl ether
0.1–5 U/ml. diaphorase
0.01–1.0 mmol/l. tetrazolium salt
0.01–10 U/ml. L(+)-tartrate dehydrogenase according to one of claims 1 or 2.

10. Process for the determination of D(+)-malate in solutions, characterised in that one converts D(+)-malate in the presence of NAD and $Mn^{2+}$ or $Mg^{2+}$ ions, with the help of L(+)-tartrate dehydrogenase according to one of claims 1 or 2, into pyruvate and $CO_2$, with the formation of NADH, and determines the resultant NADH in the usual way.

11. Reagent for the determination of D(+)-malate according to claim 10, containing
0.01–1 mol/l. buffer, pH 7.5–9,0
0.1–100 mmol/l. manganese (II) or magnesium salt
1–100 mmol/l. NAD
0.01–1 U/ml. L(+)-tartrate dehydrogenase according to one of claims 1 or 2.

## Revendications

1. Déhydrogénase de tartrate L(+), obtenable à partir de Rhodopseudomonas sphaeroides, caractérisée en ce qu'elle présente une activité de

déhydrogénase de tartrate L(+) et de déhydrogénase de malate D(+) (décarboxylant).

2. Déhydrogénase de tartrate L(+) selon la revendication 1, caractérisée en ce qu'elle présente, en plus, les propriétés suivantes:

a) NAD est nécessaire comme cosubstrat;

b) les ions $Mn^{2+}$ ou $Mg^{2+}$ sont des cations essentiels;

c) valeur optimale du pH: de 7,5 à 9,0;

d) température optimale: 50°C;

e) poids moléculaire, environ 160 000;

f) constantes de Michaelis: tartrate L(+): $2,3 \times 10^{-3}$ mol/l
malate D(+): $1,7 \times 10^{-4}$ mol/l;

g) point isoélectrique pour un pH de 5,0 à 5,2.

3. Procédé de fabrication de déhydrogénase de tartrate L(+), caractérisé en ce que l'on cultive du Rhodopseudomonas sphaeroides dans un milieu nutritif habituel qui contient, comme source de carbone, du tartrate L(+) et qu'à partir du bouillon de culture obtenu on prépare la déhydrogénase de tartrate L(+) dans les conditions habituelles.

4. Procédé selon la revendication 3, caractérisé en ce que le milieu nutritif contient du tartrate L(+) dans une concentration initiale de 20 à 50 mmol/l.

5. Procédé selon l'une des revendications 3 ou 4, caractérisé en ce que, comme souche de micro-organismes, on utilise Rhodopseudomonas sphaeroides DSM 158, Rhodopseudomonas sphaeroides DSM 159, Rhodopseudomonas sphaeroides DSM 160 ou Rhodopseudomonas sphaeroides DSM 2303.

6. Procédé de détermination de tartrate L(+) dans des solutions, caractérisé en ce que l'on fait réagir le tartrate L(+) en présence de NAD et d'ions $Mn^{2+}$ ou $Mg^{2+}$ à l'aide de déhydrogénase de tartrate L(+) selon l'une des revendications 1 ou 2 de manière à obtenir un oxalglycolate avec formation de NADH et en ce que le NADH formé est dosé d'après des procédés connus.

7. Procédé selon la revendication 6, caractérisé en ce qu'un sel de tétrazolium, un catalyseur convenable et d'autres produits auxiliaires usuels sont ajoutés et que le formazan produit est dosé par photométrie.

8. Réactif pour le dosage du tartrate L(+) selon la revendication 6, contenant
0,01 à 1,00 mol/l de tampon, pH 7,5 à 9,0
0,1 à 15 mmol/l de sel de manganèse (II) ou de sel de magnésium
2 à 50 mmol/l de NAD
0,01 à 5 U/ml de déhydrogénase de tartrate L(+) selon l'une des revendications 1 ou 2.

9. Réactif pour le dosage du tartrate L(+) selon la revendication 7, contenant
0,01 à 1,00 mol/l de tampon, pH 7,5 à 9,0
1 à 100 mmol/l de chlorure alcalin
0,01 à 100 mmol/l de sel de manganèse (II) ou de sel de magnésium
1 à 100 mmol/l de NAD
0,05 à 1,0% (v/v) d'éther de polyoxyéthylène-alkyl-aryle
0,1 à 5 U/ml de diaphorase
0,01 à 1,0 mmol/l de sel de tétrazolium
0,01 à 10 U/ml de déhydrogénase de tartrate L(+) selon l'une des revendications 1 ou 2.

10. Procédé de dosage de malate D(+) dans des solutions, caractérisé en ce qu'en présence de NAD et d'ions $Mn^{2+}$ ou $Mg^{2+}$ et à l'aide de déhydrogénase de tartrate L(+) on transforme le malate D(+) selon l'une des revendications 1 ou 2 en pyruvate et en $CO_2$ avec formation de NADH et que l'on dose comme d'habitude le NADH formé.

11. Réactif pour le dosage de malate D(+) selon la revendication 10, contenant
0,01 à 1 mol/l de tampon, pH 7,5 à 9,0
0,1 à 100 mmol/l de sel de manganèse (II) ou de sel de magnésium
1 à 100 mmol/l NAD
0,01 à 1 U/ml de déhydrogénase de tartrate L(+) selon l'une des revendications 1 ou 2.

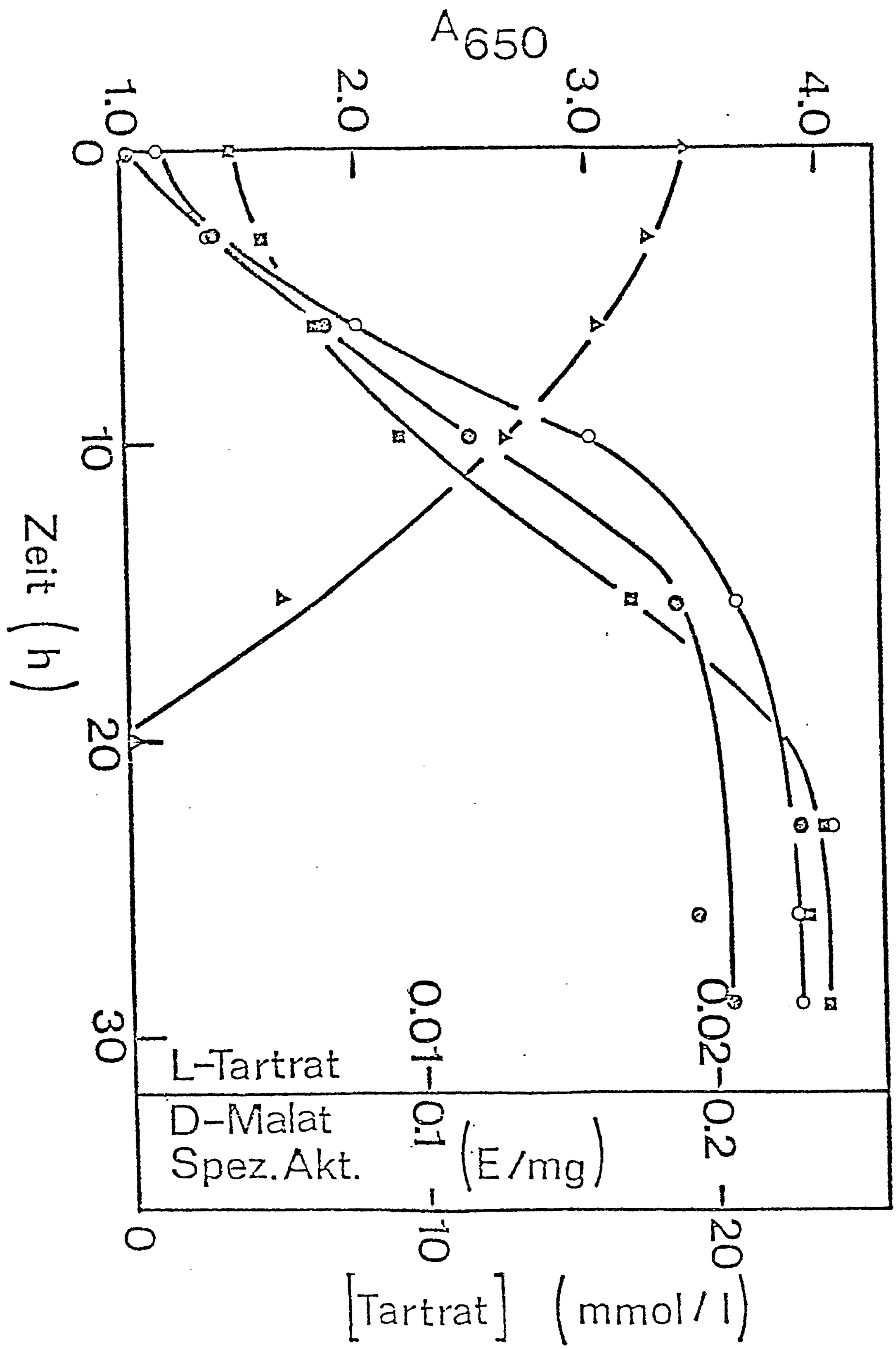
A650
1.0
2.0
3.0
4.0
0
10
20
30
Zeit (h)
L-Tartrat
D-Malat
Spez. Akt.
0.01
0.02
0.1
0.2
(E/mg)
0
10
20
[Tartrat] (mmol/l)